# EUROPEAN PATENT APPLICATION

(11) **EP 4 850 690 A1**
(43) Date of publication of application: **09.12.2026**
(21) Application number: 25180241.9
(22) Date of filing: 02.06.2025
(51) Int. Cl.: A61K 40/11, A61K 40/30, A61K 40/32, A61K 40/42, A61K 40/50, C07K 14/81

(54) **SERPINB9-OVEREXPRESSING GAMMA DELTA T CELLS FOR ALLOGENEIC CELL THERAPIES**

(71) Applicant: Captain T Cell GmbH, 12529 Schönefeld Berlin (DE)
(72) Inventor: LORENZ, Felix, 13129 Berlin (DE); SADA JAPP, Alberto, 10439 Berlin (DE); CLAUSS, Julian, 13086 Berlin (DE); EDES, Inan, 13156 Berlin (DE); STAHN, Rainer, 13125 Berlin (DE); NUSSBAUM, Nicole, 10249 Berlin (DE); HERNANDEZ REINA, Gabriela Andreina, 10557 Berlin (DE)
(74) Representative: Moré, Solveig Helga

(57) **Abstract**

The present invention relates to the field of adoptive immune cell therapy, e.g., adoptive T cell therapy. It provides γδ T cells (gamma delta T cells) engineered to overexpress serpinB9 (SB9, also known as proteinase inhibitor 9 or PI-9) and their use in treating a subject having a cancer or an infection. The invention also provides method of preparing a γδ T cell composition and methods of increasing cell viability of a γδ T cell after freezing and/or after γδ T cell activation; as well as a method for reducing immune rejection of γδ T cells after allogeneic transfer.

## Description

The present invention relates to the field of adoptive immune cell therapy, e.g., adoptive T cell therapy. It provides γδ T cells (gamma delta T cells) engineered to overexpress serpinB9 (SB9, also known as proteinase inhibitor 9 or PI-9) and their use in treating a subject having a cancer or an infection. The invention also provides method of preparing a γδ T cell composition and methods of increasing cell viability of a γδ T cell after freezing and/or after γδ T cell activation; as well as a method for reducing immune rejection of γδ T cells after allogeneic transfer.

Most T lymphocytes are αβ (alpha beta) T cells with T cell receptor (TCR) composed of two glycoprotein chains called α and β TCR chains. In contrast, γδ T cells have a TCR that is made up of one γ chain and one δ chain.

The antigenic molecules that activate γδ T cells are largely unknown. γδ T cells are peculiar in that they do not seem to require antigen processing and major-histocompatibility-complex (MHC) presentation of peptide epitopes, although some recognize MHC class Ib molecules. γδ T cells are believed to have a prominent role in lipid antigen recognition. They are of an invariant nature and may be triggered by alarm signals such as heat shock proteins (HSP).

Like other 'unconventional' lymphocyte subsets bearing invariant TCRs, such as CD1d-restricted Natural Killer cells, γδ T cells exhibit several characteristics that place them at the border between the more evolutionarily primitive innate immune system that permits a rapid beneficial response to a variety of foreign agents and the adaptive immune system, where B and T cells coordinate a slower but highly antigen-specific immune response leading to long-lasting memory against subsequent challenges by the same antigen.

γδ T cells may be considered a component of adaptive immunity in that they rearrange TCR genes to produce junctional diversity and can develop a memory phenotype. However, the various subsets may also be considered part of the innate immunity in which a specific TCR can function as a pattern recognition receptor. For example, according to this paradigm, large numbers of (human) Vγ9/Vδ2 T cells respond within hours to common molecules produced by microbes, and highly restricted intraepithelial Vδ1 T cells will respond to stressed epithelial cells bearing sentinels of danger (Wikipedia).

γδ T cells may bind to conformational epitopes, i.e. specific surface structures of proteins and possibly other molecules. For example, some γδ TCRs are known to bind to phosphoantigens and aminobisphosphonates that may be induced by metabolic changes in cells such as tumor cells. The target of a Vγ9/Vδ2 TCR is known to be a joint spatial and conformational change of CD277 (also known as BTN3A1) at the cell membrane of cancer cells. γδ T cells thus have a naturally high tumor cell killing potential. It has also been found that they can naturally infiltrate solid tumors, and that γδ T cell-rich tumors have a significantly better prognosis.

γδ T cells are also promising cells for allogeneic cell therapies, because their endogenous γδ TCR is not MHC-restricted and does not cause graft-vs-host disease (GvHD), in contrast to classical αβ T cells ^{1,2}.

Further, when γδ T cells are engineered to express alpha beta TCR, in contrast to engineered αβ T cells, there is no pairing of exogenous and endogenous alpha and beta TCR chains. γ and δ TCR chains do not pair with α and β TCR chains.

Using allogeneic T cells for adoptive T cell therapy is of high interest, because they can be used as off-the-shelf therapeutics instead of necessitating a cost- and time-intense engineering of the patients' own T cells. Because of scalability, the cost per dose would be about 10 times lower. Further, progressing patients may be immediately treated, and there is a good fitness of the T cells - often in contrast to patients' T cells, which may already be affected by the disease of the patient and/or previous treatments. Manufacturing failures for specific patients can also be avoided, and the cells can be more easily standardized.

Allogeneic CAR-γδ T cells and unmodified γδ T cells have already been employed in clinical trials and showed outstanding safety ^{3.4}. In addition, γδ T cells can be easily obtained from the peripheral blood of healthy donors and engineered to express cancer-targeting receptors. The inventors have previously shown that γδ T cells engineered with a cancer-specific αβ TCR (CTC127, WO 2022/243514 A1) and a CD8 co-receptor can recognize and kill cancer cells (WO 2025/027111 A1).

Some challenges still need to be tackled in order to fully realize the potential of allogeneic γδ T cells.
1) They are very susceptible to activation-induced cell death (AICD) ^{5,6}, which may limit their in vitro expansion during manufacturing and in vivo efficacy upon repeated antigenic stimulation.
2) They are poorly resistant to cryopreservation ⁷, an essential step for an off-the-shelf allogeneic cell therapy product.
3) As with other allogeneic therapies, γδ T cells may also face allogeneic rejection by the host's immune system, which can limit the persistence of the cell product in the patient's body and thereby curtail clinical responses ⁸.

In light of this, the inventors addressed the problem of providing more robust cells suitable for allogeneic cell therapies and methods of preparing them. This problem is solved by the present invention, in particular, by the subject-matter of the claims. The invention provides a γδ T cell comprising a nucleic acid encoding SerpinB9, wherein the SerpinB9 has at least 80% sequence identity to wt human SerpinB9 of SEQ ID NO: 1 and is capable of reducing granzyme B activity, and wherein the γδ T cell expresses said SerpinB9, wherein the nucleic acid encoding SerpinB9 is operably linked to a heterologous promotor and is DNA or wherein the nucleic acid encoding SerpinB9 is an mRNA having a heterologous cap. Preferably, SerpinB9 is operably linked to a heterologous promotor and is DNA.

Accordingly, the inventors propose a novel toolbox technology that improves robustness of γδ T cells.

SerpinB9 (SB9) is the only known inhibitor of granzyme B. It is naturally expressed by cytotoxic T and NK cells and protects against self-inflicted damage ⁹⁻¹³. Binding of SB9 and granzyme B is irreversible and occurs with a 1 to 1 stoichiometry. Thus, when granzyme B production and secretion is increased (e.g. upon antigenic stimulation) the SB9 stocks can be exhausted and the cell can become susceptible to granzyme B-mediated cell death ^{10,13,14}. Leakage of granzyme B from cytotoxic vesicles occurs after a freeze-thaw cycle and can cause apoptosis, which can greatly reduce cell recovery after cryopreservation ¹⁵. As discussed above, γδ T cells are particularly susceptible to AICD, to bad recovery after cryopreservation and to allogeneic rejection.

The role of SB9 in this, in particular, expression levels and effects of SB9 on γδT cells, are largely unknown. The inventors show that overexpression of SB9 in γδ T cells, e.g., in therapeutic allogeneic αβTCR-engineered γδ T cells, increases their robustness and enables efficient expansion, cryopreservation and protection against allogeneic rejection.

The SerpinB9 can be wt (wildtype) human SB9 of SEQ ID NO: 1. It can also be a variant thereof, e.g. a variant resistant to inactivation by reactive oxygen species. For example, the SB9 can have C341S and/or C342S substitutions. This increases its resistance to inactivation by reactive oxygen species. It can also be a variant having an increased affinity to a caspase, e.g., caspase 1, 2, 5, 6, 7, 8 and/or 10, some of which are involved in Fas-mediated and TNFα-mediated apoptotic pathways. For example the SB9 can have a E340D substitution (SB9(CAS)), which has been shown to lead to increased affinity to a caspase, e.g., e.g., caspase 1, 2, 5, 6, 7, 8 and/or 10. Such SB9 variants may be more effective in particular in increasing robustness of the cells *in vivo* after transfer to the patient, as Fas-mediated killing plays a role in downregulation of immunological responses in the tumor microenvironment. The SB9 can also have C341S and/or C342S and E340D substitutions, optionally, all three. The SB9 variant carrying the three amino acid substitutions (E340D, C341S, and C342S) (SB9(ROSCAS) SEQ ID NO: 4) has not been previously described, to our knowledge, and is particularly interesting, as it can potentially be resistant to ROS inactivation and have increased affinity to caspases.

Amino acid sequences for such preferred variants of SB9 are, e.g., SEQ ID NO: 2, 3 or 4. For comparison, SB9(NEG) comprising SEQ ID NO: 5, SB9(NEG) comprises a T327R mutation and is a non-functional SB9 variant that is not capable of reducing granzyme B activity. It was in some experiments used as a negative control. Functional SB9 used according to the invention preferably does not comprise 327R. Rather, it preferably comprises T in position 327. If not mentioned otherwise, a reference to SB9 in the context of the invention refers to a functional SB9 capable of reducing granzyme B activity.

Although it was previously also observed that SB9(WT) overexpression increased donor T cell resistance of αβ T cells expressing a chimeric antigen receptor (CAR) to recipient-mediated cell cytotoxicity *in vitro,* it was insufficient to protect donor T cells against recipient T cell-mediated allorejection *in vivo.* By contrast, SB9(CAS), which interacts with granzyme B and several caspases, greatly enhanced alloprotection, cell expansion, and antitumor efficacy of αβ T cells expressing a chimeric antigen receptor (CAR) both *in vitro* and *in vivo* ²⁶.

Still, in the context of the γδ T cells, the present inventors showed that wt human SB9 improves resistance to AICD. SB9 advantageously further improves cryoresistance and resistance to allogeneic killing. It is thus possible to use wt SB9 overexpression in γδ T cells to render them more robust. This avoids the risk of immunologic reactions to genetically engineered variants of SB9.

In the context of human cells, human SB9 is preferably used. However, it is also possible to use, e.g., mouse SB9, e.g., in the context of mouse cells. For example, mouse SB9(WT) can be used or its respective CAS or ROS variants may also be employed.

The nucleic acid encoding SB9 may, e.g., have SEQ ID NO: 6 (human wt SB9). It may also be a codon-optimized variant thereof (e.g., SEQ ID NO: 7). The nucleic acid sequence may comprise one or more exons. Variants of SB9 may be encoded, e.g., by any of SEQ ID NO: 8-10. SEQ ID NO: 11 encodes the comparative SB9(NEG).

In a preferred embodiment, the nucleic acid encoding SB9 is operably linked to a heterologous promotor. In one embodiment, such a nucleic acid, including the EF1α promotor and SB9(WT), has SEQ ID NO: 11. Other SB variants, e.g., as disclosed herein, can be substituted for the wildtype. The nucleic acid typically is DNA in this context. Operably linked means that activation of the promotor leads to transcription of the encoded SB9. A heterologous promotor is not operably linked to SB9 in non-engineered human cells. The promotor is functional in human cells, in particular, in human γδ T cells. Optionally, the promotor is only functional in immune cells, in particular, in T cells, but not in other human cells, preferably only in γδ T cells. The promotor can be a constitutive promotor. It can also be an inducible promotor, which can further improve the safety of the transfected cells. Preferably, it is a strong promotor to ensure strong overexpression. The promoter can be a non-viral promoter or a viral promoter. The inventive recombinant expression vectors can be designed for either transient expression, for stable expression, preferably, for stable expression. For example, the promotor can be selected from the group comprising human EF1α, PGK, beta actin, IF4A1, GAPDH, GRP78, HSP70, beta kinesin, ubiquitin B, CD45, MPSV, CMV promotor or a long terminal repeat promotor. CD45 is an exemplary promotor specifically active in immune cells. Preferably, the promotor is EF1α.

The nucleic acid may, e.g., be a viral vector, a transposon, a vector suitable for CRISPR/CAS based recombination or a plasmid suitable for in vitro RNA transcription. The nucleic acid of the invention preferably is a vector. The vector may be an expression vector suitable for expression in a γδ T cell, preferably, a human γδ T cell. The vector may be a viral vector, e.g. a retroviral, in particular gamma-retroviral or lentiviral vector. Examples of suitable expression vectors include the retroviral vector MP71. The expression vector comprises regulatory sequences, such as transcription and translation initiation and termination codons. Furthermore, the vector of the invention may include one or more marker genes, which allow for selection of transduced or transfected hosts. Preferably, there is not antibiotic resistance marker.

The vector may also encode for other proteins, e.g., in other expression cassettes or separated from the SB9 encoding sequence by a self-cleaving peptide sequence, e.g., a P2A sequence (SEQ ID NO: 13). For example, the heterologous promotor may be operably linked to a sequence encoding at least one of a TCR alpha chain or a TCR beta chain (optionally, the TCR beta chain), linked to the other of the TCR alpha chain or a TCR beta chain via a self-cleaving peptide sequence, e.g., a P2A sequence, linked to a SB9 encoding sequence via a self-cleaving peptide sequence, e.g., a P2A sequence. Alternatively, the heterologous promotor is operably linked to a sequence encoding at least one of a TCR alpha chain or a TCR beta chain (optionally, the TCR beta chain), linked to a SB9 encoding sequence via a self-cleaving peptide sequence, e.g., a P2A sequence, linked to the other of the TCR alpha chain or a TCR beta chain via a self-cleaving peptide sequence, e.g., a P2A sequence. In a further embodiment, the heterologous promotor is operably linked to a SB9 encoding sequence linked to a sequence encoding at least one of a TCR alpha chain or a TCR beta chain (optionally, the TCR beta chain), via a self-cleaving peptide sequence, e.g., a P2A sequence, linked to the other of the TCR alpha chain or a TCR beta chain via a self-cleaving peptide sequence, e.g., a P2A sequence. The vector may also encode for other proteins capable of improving growth or resistance to inactivating stimuli, e.g., separated from the SB9 encoding sequence by a self-cleaving peptide sequence such as a P2A sequence. Such a protein could for example be a SWITCH receptor capable of converting a TGFbeta signal into a positive stimulus for the cell, e.g., as disclosed in WO 2025/036957 A1.

Alternatively, the nucleic acid encoding SB9 is an mRNA having a heterologous cap. Cap structures play an important role, e.g., in initiation of translation, nuclear export, preventing degradation and thus in stabilizing the mRNAs. The cap is a specially altered nucleotide on the 5' end of transcripts such as precursor mRNA. In eukaryotes, normally, the 5' cap (cap-0), found on the 5' end of an mRNA molecule, consists of a guanine nucleotide connected to mRNA via an unusual 5' to 5' triphosphate linkage. This guanosine is methylated on the 7 position. It is referred to as a 7-methyl-guanylate cap, abbreviated m⁷G. The Cap-0 is the base cap structure, however, the first and second transcribed nucleotides can also be 2' O-methylated, leading to the Cap-1 and Cap-2 structures, respectively (Wikipedia). The nucleic acid of the invention does not comprise such a homologous cap. Accordingly the mRNA of the invention is an artificial mRNA that is modified - compared to a natural DNA - to be particularly stable. A heterologous cap is a cap structure that does not occur in nature in the cells of the species from which the γδ T cell is derived, typically, a human cell. For example, the heterologous cap is selected from the group comprising an anti-reverse cap analog (ARCA), a two-headed cap analog, a locked nucleic acid modified cap or a cap comprising a chemical modification of the phosphate bridge comprising a tetraphosphate structure and/or a substitution of O by S. An anti-reverse cap analog (ARCA) can be modified in the 3' position of the m⁷G, such as m₂^{7,3'-O}GpppG or m⁷d^{3'}GpppG. A two-headed cap analog does not depend on the direction it is included in tha mRNA. It can e.g. be m⁷Gppprn⁷G. A locked nucleic acid modified cap can e.g., be m⁷, ^{LNA}GpppG. Increased stability and increased affinity for the translation factor elF4E can also be obtained by chemical modification of the phosphate bridge comprising a tetraphosphate structure or a substitution of O by S. The analog can e.g., be 1Sp analog or a 2S4p analog.

The γδ T cell of the invention preferably further expresses a TCR construct, preferably, a MHC class I restricted αβ TCR construct, and a CD8 or CD4 co-receptor, preferably, a CD8 co-receptor. The inventors have shown that co-expression of a CD8 co-receptor, in particular, of a CD8 alpha beta co-receptor and a MHC class I restricted TCR construct endows γδ T cells with the functions typically seen in CD8⁺ cytotoxic T cells, e.g., IFN-γ production upon co-culture with tumor cells

expressing the relevant epitope and MHC I, as well as cytotoxic functions. In contrast, γδ T cells expressing only the TCR without the CD8 co-receptor fail to elicit the full breadth of responses of the γδ T cells against the targeted solid tumor cells. Accordingly, the γδ T cells of the invention co-expressing CD8 co-receptor and a MHC class I restricted TCR construct, in particular, from the nucleic acid of the invention as described herein, can advantageously be used in adoptive T cell therapy, e.g., in allogeneic adoptive T cell therapy. In contrast to CAR T cells, T-cells expressing MHC class I restricted TCR can target intracellular antigens, such as tumor antigens. This also applies for intracellular solid tumor antigens, such as MAGE, e.g., MAGE-A4. Downregulation of cell surface expression is thus not sufficient for evading the action of such T cells. Optionally the TCR is a TCR disclosed in WO 2024/042202 A1 targeting MAGE-A4.

If the γδ T cells of the invention express a CD4 co-receptor, they typically express a MHC class II restricted TCR construct. Some TCRs are also co-receptor independent.

The inventors have additionally shown that γδ T cells can naturally sense HLA (Human leukocyte antigen / MHC class I) loss by tumor cells. For example, the HLA-negative cell line K562 is recognized by non-engineered γδ T cells, probably though innate immune function. This may provide an additional mode of action if tumor cells try to escape through downregulation of HLA.

Preferably, the donor shares with the patient that is to be treated the MHC, preferably, the MHC I, to which the TCR with which the γδ T cell is engineered is restricted, and, most preferably, the donor is haploidentical for this MHC. For example, the donor can have two alleles of HLA-A*01:01, which is the second most frequent HLA allele in Europe. It is also the HLA to which CTC127, a TCR recognizing a MAGE-A4 epitope (WO 2022/243514 A1), is restricted.

This, together with the fact that γδ T cells do not express high levels of MHC II molecules, already largely reduces the risk of host-versus graft reactions. Optionally, other HLA-molecules can be downregulated, e.g., by inhibitory RNA or knocked out by methods such as CRISPR/Cas engineering. Donors can be selected who are also haploidentical for one or, preferably, both other HLA molecules. Further, libraries of γδ T cells having the most common HLA-types, preferably, in haploidentical form, can be provided, so that suitable γδ T cells for a particular patient can be selected.

The co-receptor preferably is CD8. As disclosed in WO 2025/027111 A1, it is advantageously encoded by a nucleic acid encoding a CD8 αβ co-receptor, wherein α chain and β chain are not covalently linked, and a TCR α chain construct and a TCR β chain construct, wherein the nucleic acid comprises an operon capable of mediating expression of the CD8 α and CD8 β chain and a TCR α chain construct and a TCR β chain construct under the control of one promotor,
wherein the nucleic acid segment encoding the CD8 β chain is more proximal to the promotor than the nucleic acid segment encoding the CD8 α chain,
and wherein at least one nucleic acid segment encoding a CD8 α chain or CD8 β chain is between the nucleic acid segment encoding one of the TCR α chain construct and the TCR β chain construct and the nucleic acid segment encoding the other of the TCR α chain construct and the TCR β chain construct.

In one embodiment, SB9 is also encoded on the nucleic acid encoding a CD8 αβ co-receptor, wherein α chain and β chain are not covalently linked, and a TCR α chain construct and a TCR β chain construct, wherein the nucleic acid comprises an operon capable of mediating expression of the CD8 α and CD8 β chain and a TCR α chain construct and a TCR β chain construct under the control of one promotor,
wherein the nucleic acid segment encoding the CD8 β chain is more proximal to the promotor than the nucleic acid segment encoding the CD8 α chain,
and wherein at least one nucleic acid segment encoding a CD8 α chain or CD8 β chain is between the nucleic acid segment encoding one of the TCR α chain construct and the TCR β chain construct and the nucleic acid segment encoding the other of the TCR α chain construct and the TCR β chain construct. Such a nucleic acid would thus comprise at least five protein-encoding segments, i.e., in addition to the segment encoding the SB9, four segments: the segment encoding the CD8 β chain, the nucleic acid segment encoding the CD8 α chain, the nucleic acid segment encoding the CD8 α chain or the nucleic acid segment encoding the CD8 β chain. The nucleic acid segment encoding SB9 in this context can be situated between the promotor and the fist protein-encoding nucleic acid segment. The nucleic acid segment encoding SB9 can alternatively be situated between the first and the second protein-encoding nucleic acid segments, between the second and the third protein-encoding nucleic acid segments, between the third and the fourth protein-encoding nucleic acid segments and distal of the four other protein-encoding nucleic acid segments (relative to the promotor). All of these segments can be separated from each other by a cleavage site, e.g., P2A-element.

For example, the promotor can be operably linked to the nucleic acid segment encoding the SB9, which is, via a nucleic acid segment encoding a cleavage site, linked to the nucleic acid segment encoding the CD8 beta chain, which is, via a nucleic acid segment encoding a cleavage site, linked to the nucleic acid segment encoding the TCR beta chain construct, which is, via a nucleic acid segment encoding a cleavage site, linked to the nucleic acid segment encoding the CD8 alpha chain, which is, via a nucleic acid segment encoding a cleavage site, linked to the nucleic acid segment encoding the TCR alpha chain construct.

Alternatively, the promotor can be operably linked to the nucleic acid segment encoding the CD8 beta chain, which is, via a nucleic acid segment encoding a cleavage site, linked to the nucleic acid segment encoding the SB9, which is, via a nucleic acid segment encoding a cleavage site, linked the nucleic acid segment encoding the TCR beta chain construct, which is, via a nucleic acid segment encoding a cleavage site, linked to the nucleic acid segment encoding the CD8 alpha chain, which is, via a nucleic acid segment encoding a cleavage site, linked to the nucleic acid segment encoding the TCR alpha chain construct.

Alternatively, the promotor can be operably linked to the nucleic acid segment encoding the CD8 beta chain, which is, via a nucleic acid segment encoding a cleavage site, linked to the nucleic acid segment encoding the TCR beta chain construct, which is, via a nucleic acid segment encoding a cleavage site, linked to the nucleic acid segment encoding the SB9, which is, via a nucleic acid segment encoding a cleavage site, linked to the nucleic acid segment encoding the CD8 alpha chain, which is, via a nucleic acid segment encoding a cleavage site, linked to the nucleic acid segment encoding the TCR alpha chain construct.

Alternatively, the promotor can be operably linked to the nucleic acid segment encoding the CD8 beta chain, which is, via a nucleic acid segment encoding a cleavage site, linked to the nucleic acid segment encoding the TCR beta chain construct, which is, via a nucleic acid segment encoding a cleavage site, linked to the nucleic acid segment encoding the CD8 alpha chain, which is, via a nucleic acid segment encoding a cleavage site, linked to the nucleic acid segment encoding the SB9, which is, via a nucleic acid segment encoding a cleavage site, linked to the nucleic acid segment encoding the TCR alpha chain construct.

Alternatively, the promotor can be operably linked to the nucleic acid segment encoding the CD8 beta chain, which is, via a nucleic acid segment encoding a cleavage site, linked to the nucleic acid segment encoding the TCR beta chain construct, which is, via a nucleic acid segment encoding a cleavage site, linked to the nucleic acid segment encoding the CD8 alpha chain, which is, via a nucleic acid segment encoding a cleavage site, linked to the nucleic acid segment encoding the TCR alpha chain construct, which is, via a nucleic acid segment encoding a cleavage site, linked to the nucleic acid segment encoding the SB9.

Alternatively, the TCR construct can also be a αβ TCR construct restricted to MHC II and the co-receptor can be a CD4 coreceptor. In one embodiment, both CD4+ with an MHC II-restricted TCR and CD8+ T cells with an MHC I-restricted TCR can be combined, wherein the TCRs both recognize epitopes from the same antigen.

In still another embodiment, the γδ T cell of the invention does not express a TCR construct restricted to MHC I or II. Instead, it expresses a chimeric antigen receptor (CAR). CARs are chimeras of the antigen-binding domains of antibodies capable of recognizing cell surface antigens combined with TCR domains. T cells engineered to express the CAR thus target cells expressing the antigen to which the CAR binds, irrespective of any HLA restriction. For example, CAR T-cells targeting B-cell antigens have proven successful, demonstrating the potency of adoptive T-cell therapy. Recently, clinical studies of adoptive T-cell therapy (ATT) using chimeric antigenic receptors gene-transfer against the B-cell antigen CD19 have achieved remarkable success in 40-70% of patients with B-cell lymphomas or leukemias, and the therapy been designated as "breakthrough cancer therapy". CARs can thus e.g., target B-cell lineage antigens such as CD19, CD20 and CD22.

A γδ T cell of the invention expressing a CAR may further express a CD4 or a CD8 co-receptor. Compositions comprising such cells may also comprise a mixture of cells comprising CD4 and CD8 co-receptors.

A CAR may e.g. be a first generation CAR, comprising a CD4 extracellular domain and a CD3ζ intracellular domain, a second generation CAR further comprising at least one co-stimulatory intracellular domain e.g. derived from CD28 or 4-1BB, or a third generation CAR comprising two co-stimulatory domains, e.g., from CD28 and 4-1BB. Optionally, it may further comprise a cytokine inducer domain. For example, T cells redirected for universal cytokine-mediated killing (TRUCKs) that express, e.g., IL-7, IL18 and/or CCL19 can be generated this way.

The CARs may be a CAR from a CAR-T therapy approved to the marked in the context of αβ T cells, e.g., one of the CARs of Axi-cel, Tisa-cel, Liso-cel, Brexu-cel (all targeting CD19), or Ide-cel or Cilta-cel (targeting BCMA).

The antigen targeted by the TCR or CAR preferably is an epitope from a cancer-specific or cancer-associated antigen (e.g., MAGE-A1, MAGE-A4, PRAME, NY-ESO-1, KRAS, CD19, CD70, BCMA, B7-H2, CLDN6) or an infectious agent, e.g., a virus such as HIV, EBV or HPV, Ebola, SARS-CoV1, SARS-CoV2 or MERS or a bacterium, e.g., an antibiotic-resistant bacterium. As γδ T cells are particularly suited for targeting cancer cells, the antigen preferably is a cancer antigen, e.g., a neoantigen. For example, the antigen may be an antigen overexpressed or exclusively expressed by a solid tumor.

In one embodiment, γδ T cell of the invention further expresses a kill switch. A kill switch, also designated safety or suicide switch, enables the control of treatment-related adverse events, for instance, cytokine release syndrome (CRS) or graft-vs-host disease (GvHD), by disabling T cells with exogenous agents. Kill switches can deplete the T cells expressing the kill switch by inducing apoptosis, complement-dependent cytotoxicity (CDC) or antibody-dependent cellular cytotoxicity (ADCC) upon administration of an exogenous agent. Switchable T cells are engineered by additional transduction of genes that encode easily targetable surface antigens or inducible intracellular effectors. As soon as the incorporated gene is expressed, the cells become susceptible to specific pharmaceuticals and can be depleted if necessary.

For example, the T cell can express a protein or domain thereof that can be targeted by an antibody. In a preferred embodiment, the T cell expresses EGFR deficient in signaling on its surface, optionally, wherein the EGFR is a truncated EGFR (tEGFR). This can be targeted by a specific antibody, e.g., cetuximab. This enables removal of the T cell via ADCC or CDC. Optionally, the EGFR, e.g., tEGFR is expressed in tandem to the SB9. The EGFR can be expressed preferably, from the same vector as the SB9, e.g., linked to it by a self-cleaving peptide such as P2A element. Expression as a fusion protein, with SB9 on the cytosolic side, is also possible.

The inclusion of the RQR8 suicide gene is an analogous approach to controlling T cells after therapeutic administration. The RQR8 gene encodes a cell surface protein combining epitopes derived from CD20 and CD34 antigens. This strategy enables T cell depletion via CDC or ADCC following the administration of monoclonal antibody rituximab.

Alternatively, the cell may be engineered to express inducible caspase 9. If required, administration of an agent such as AP1903 (rimiducid) may then elicit dimerization of inducible caspase 9 which induces apoptosis., Another suicide gene that can be utilized as a safety switch in T cells of the invention is herpes simplex virus thymidine kinase (HSV-TK) Mut 2 gene, the product of which is targeted by the prodrug ganciclovir (GCV). HSV-TK converts GCV into GCV-triphosphate, a competitive inhibitor of deoxyguanosine incorporation into DNA that causes cell death by disrupting the replication process ²⁷.

In one embodiment, expression or stabilization of SB9 can be regulated.

Accordingly, the cell may be engineered to express SB9 under an inducible promotor. SB9 can also be expressed in an inducible system. For example, expression can be induced by tetracyline (tet-on), beta-galactosidase, doxycycline, rapamycin, rapalogues, or veledimex.

SB9 could also be fused to a drug responsive domain (DRD) tag, which can control the stability of the protein ³⁰. The DRD, e.g., carbonic anhydrase 2 (CA2) is stabilized in the presence of a small molecule ligand, e.g., acetazolamide (ACZ) or celecoxib. ACZ is orally bioavailable and FDA approved. In the absence of the small molecule ligand, the DRD assumes a disordered conformation that is recognized by the quality control mechanisms of the cell, resulting in degradation of the fusion protein by the proteasome.

Inducible expression or inducible stabilization of SB9 may be an important safety feature. In case killing of the transgenic, SB9-transfected cells is intended in vivo, e.g., through a kill switch as explained herein, enduring presence of SB9 may delay death of the cells. This effect can be counteracted by using an inducible system, e.g., an inducible promotor controlling SB9 expression or inducible stabilization of SB9.

Alternatively, expression of SB9 can be downregulated in case of need, e.g., by expression of double stranded RNA capable of inducing RNAi (miRNA or siRNA) under the control of an inducible promotor, e.g., as disclosed herein. This can be encoded on the nucleic acid encoding SB9.

Thus, in particular if the cells express a kill switch, it may improve safety if expression or stabilization of SB9 can be regulated.

Optionally, the γδ Tcell of the invention further expresses a SWITCH receptor, wherein the switch receptor is capable of transforming a signal provided by TGF-β into a co-stimulatory signal. The SWITCH receptor is thus distinct from the kill switch mentioned above. The switch receptor may e.g., comprise
a) a TGFβ - binding domain that is an scFv,
b) a hinge domain,
c) a transmembrane domain,
d) an intracellular costimulatory signaling domain, wherein, after binding of the switch receptor to TGFb, the intracellular costimulatory signaling domain is not capable of activating a T cell in the absence of further stimulation. Preferred switch receptors are disclosed in WO 2025/027111 A1, e.g., in Table 1 thereof. SR01, SR02, SR03 and SR04 were shown to be most effective, in particular SR02.

The invention also provides a composition comprising the γδ T cell of the invention and a suitable solvent. The solvent typically is an aqueous solvent, e.g., a buffer, a cell culture medium or a freezing medium. A freezing medium may e.g., comprise cell culture medium (e.g., 4Cell^{®} Nutri-T GMP medium) and a cryoprotectant such as dimethyl sulfoxide (DMSO), e.g., 10% DMSO or glycerol. Exemplary freezing media are, e.g., Recovery Cell Culture Freezing Medium or Synth-a-Freeze Cryopreservation medium, (both ThermoFisher Scientific) or CryoStor^{®} (STEMCELL Technologies Inc.) Preferably, the medium comprises an isotonic solution suitable for administration to humans..

For administration to humans, the composition does not comprise any proteins derived from non-human animals. It may also be free of antibiotics, antimycotics, hormones, growth factors, serum and/or proteins.

It is one of the advantages of the invention that the cells expressing SB9 are more resistant to cryopreservation. Thus, the composition of the invention can be frozen. Freezing can be in liquid nitrogen or at - 20°C, preferably, in liquid nitrogen. This allows long-term preservation and storage of γδ T cells of the invention. They can be stored, e.g., for one day, two, three, four, five, six, seven, eight, nine or ten days or for 1-500 weeks, e.g., for 1-36 months or for 12-24 months. The invention also provides a library of frozen compositions comprising γδ T cells of the invention, e.g., each expressing a different TCR. For example, each composition can comprise γδ T cells of the invention expressing TCR targeting an epitope from the same tumor antigen on a different MHC, preferably, MHC class I.

The composition can also be a thawed composition. After thawing, the cells, still in their freezing medium (e.g. CryoStor^{®}), may be directly administered to the patient, or they may be washed and resuspended in a medium suitable for administration to a human patient.

The composition preferably is a pharmaceutical composition. The γδ T cell of the invention or the composition of the invention may be for use in treating a subject having a cancer or an infection, preferably, a cancer, by adoptive T cell therapy, wherein the γδ T cell is an allogeneic cell. If the T cell is in a frozen composition, the composition is thawed before administration to the subject.

The invention also provides a method for treating a subject having a cancer or an infection, preferably, a cancer, by adoptive T cell therapy, wherein the γδ T cell is an allogeneic cell, comprising administering the γδ T cell of the invention or the composition of the invention to said subject.

The pharmaceutical compositions of the invention typically are for intravenous administration. They may further comprise pharmaceutically acceptable carriers such as buffers, e.g., physiological saline or PBS. They may further comprise an excipient, such as a stabilizer, for example SPGA, DMSO, carbohydrates (e.g. sorbitol, mannitol, starch, sucrose, glucose, dextran), a protein such as albumin or casein, or protein containing agents such as bovine serum or skimmed milk. Preferably, it does not comprise non-human proteins.

The γδ T cells can be, e.g., administered to a patient in a concentration of 1 x 10⁵ - 1 x 10⁹ cells per kg. About 1 x 10⁶ - 2 x 10¹¹ cells may be administered to a patient as a single dose. These parameters may be adapted by the medical practitioner, depending, e.g., on the patients age, sex, body weight and medical condition, e.g., as known in the art.

Pharmaceutical compositions and kits of the present invention may be used in combination with other agents, in particular, for treatment of cancer, with other anti-cancer agents. For example, other anti-cancer agents may be TCR-engineered αβ T cells or γδ T cells expressing TCRs specific for other antigens expressed by tumors (e.g. MAGE-A4, MAGE-A3, NY-ESO or others may be co-expressed by a tumor), checkpoint inhibitors or other immunotherapies as well as antibodies, small molecule inhibitors or other types of reagents.

One preferred medicinal use of the invention is immune therapy, preferably adoptive T cell therapy. The product and methods of the invention are particularly useful in the context of adoptive T cell therapy. The administration of the compounds of the invention can for example involve the administration, e.g., infusion of γδ T cells of the invention into said patient.

The subject preferably is a human patient, e.g., a patient having a solid tumor. The solid tumor can be, e.g., melanoma, head and neck cancer, breast cancer, ovarian cancer, lung cancer, epithelial cancer, pancreas cancer, colorectal cancer, synovial sarcoma, or multiple myeloma. Optionally, the cancer is a MAGEA4-associated cancer. For example, if the TCR expressed by the cell of the invention targets an epitope from MAGE-A4, it may be for use in the treatment of a subject having a cancer expressing MAGEA4 e.g., head and neck cancer, lung cancer, colorectal cancer, and synovial sarcoma. The cancer can also be a PRAME-, or MAGEA1-, associated cancer. The pharmaceutical composition of the invention is for use in the treatment of a patient expressing an MHC in the context of which the respective TCR recognizes the respective epitope. Alternatively, the pharmaceutical composition of the invention is for use in the treatment of a patient whose cancer expresses the antigen recognized by a CAR. In the context of CAR, the MHC haplotype is irrelevant.

The γδ T cell of the invention can be, e.g., a mouse cell, but preferably it is a human cell. Advantageously, it can thus be used for the treatment of a human patient. The γδ T cell of the invention can be derived from said patient, but, typically, it is an allogeneic cell. As γδ T cells do not recognize allogeneic MHC molecules, allogeneic cells can be safely used without the risk of graft versus host disease. The γδ T cell can thus be derived from a donor, e.g., a healthy donor. It is typically isolated from peripheral blood of the donor. γδ T cells can be, e.g., derived from PBMC or T cells isolated therefrom. They can be enriched by antibodies against the gamma and/or delta TCR chains, and/or by depletion of alpha beta T cells and/or by stimulation with bisphosphonates and/or by stimulation with anti-CD3 antibodies ^{28, 29}.

The inventors could show that a 100fold expansion of TCR-engineered γδ T cells, e.g., from PBMC of a healthy donor, was possible. Protocols for γδ T cell expansion that may be used are known in the art, e.g, ²⁸, ²⁹, US 2003219463 A1, or WO2019/104269 A1. The γδ T cells of the invention, which carry an αβ TCR construct, can be stimulated with anti-CD3 antibodies which stimulate all subsets of γδ T cells. Alternatively, the Vγ9Vδ2 subset can be specifically stimulated using isopentenyl pyrophosphate (IPP), bromohydrin pyrophosphate (BrHPP) or bisphosphonates (e.g. zoledronate). γδ T cells can also be generated from iPSC, e.g., human iPSC. Alternatively, engineered γδ T cells expressing the co-receptor and an MHC class I restricted TCR can be expanded antigen-specifically, e.g., with cells expressing the target antigen, and/or cells coated with the cognate peptide.

The invention provides a method of increasing cell viability after γδ T cell activation, comprising transfecting a γδ T cell with a nucleic acid encoding SerpinB9, thus generating a γδ T cell of the invention. The invention provides a method of increasing cell viability of a γδ T cell after freezing, comprising transfecting a γδ T cell with a nucleic acid encoding SerpinB9, thus generating a γδ T cell of the invention. The invention also provides a method for reducing immune rejection of γδ T cells after allogeneic transfer, comprising transfecting a γδ T cell with a nucleic acid encoding SerpinB9, thus generating a γδ T cell of the invention. Preferably, the invention provides a method of increasing cell viability of a γδ T cell after freezing; and of increasing cell viability after γδ T cell activation and for reducing immune rejection of γδ T cells after allogeneic transfer, comprising transfecting a γδ T cell with a nucleic acid encoding SerpinB9, thus generating a γδ T cell of the invention.

The invention is further directed to a method of preparing a γδ T cell composition, comprising
a) providing a γδ T cell,
b) transfecting the γδ T cell with a nucleic acid encoding SerpinB9, thus generating a γδ T cell of any of the invention,
c) freezing a composition comprising said T cell,
d) thawing the composition comprising said T cell, and
e) formulating the composition for administration to a human subject,
wherein, optionally, the method further comprises restimulating the T cell composition. Restimulation can be between steps a) and c) and/or b) and c) and/or d) and e). As restimulation after thawing complicates the work to be done at the place where the patient is to be treated, the method preferably does not comprise restimulating the T cell composition after thawing. Preferably, restimulation is thus carried out before freezing, in particular, between steps b) and c).

In step a), the γδ T cell can be obtained, e.g., from human PBMC. The PBMC are preferably obtained from a healthy human donor. They may be collected by leukopheresis or from a blood donation. The γδ T cells are typically isolated from the blood sample or from the PBMC, e.g., as disclosed herein or in the references cited herein. The γδ T cells used for step b) are typically, at least 80% γδ T cells, preferably, at least 90% γδ T cells, e.g., at least 95% γδ T cells or at least 99% γδ T cells.

The transfection in step b) comprises contacting the γδ T cell with a nucleic acid encoding SB9, e.g., a vector as disclosed herein. The γδ T cells are typically expanded before freezing. It is advantageous to initiate said expansion after step b), because the expression of SB9 makes them more resistant to activation-induced cell death. The expansion is initiated by restimulation, e.g., as disclosed herein.

Freezing typically involves removing the cell culture medium and resuspension in a freezing medium, or addition of a cryoprotectant to the cell culture medium. It can also involve gradually lowering the temperature of the composition, e.g., after said gradual lowering, freezing in liquid nitrogen.

Thawing is typically done by raising the temperature, optimally, to 37°C, e.g., by immersion in a water bath at that temperature, and then, optionally, exchanging the medium to remove the cryoprotectant. The cells can then be formulated for administration to a human subject. In particular, they can be resuspended in a biocompatible medium. The cells can be ready to use at that time point. Alternatively, after thawing, they can be restimulated again for further activation and/or expansion before they are formulated for administration to the human subject. In a further embodiment, the cryoprotectant is pharmaceutically acceptable for a human subject at the concentration used, and the thawed composition can, after raising the temperature, e.g., to 37°C, be directly administered to the human subject.

In the context of the present invention, "a" is understood to mean "one or more" unless expressly stated otherwise. "About" is to be understood as +/- 10%.

The present invention is further illustrated in the following examples with reference to the accompanying figures and sequences, nevertheless, without being limited thereto. For the purposes of the present invention, all references as cited herein are incorporated by reference in their entirety.
SEQ ID NO: 1 SB9(WT) amino acids
SEQ ID NO: 2 SB9(ROS) amino acids
SEQ ID NO: 3 SB9(CAS) amino acids
SEQ ID NO: 4 SB9(ROSCAS) amino acids
SEQ ID NO: 5 SB9(NEG) amino acids
SEQ ID NO: 6 SB9(WT) nucleic acid
SEQ ID NO: 7 SB9(WT) nucleic acid, codon-optimized
SEQ ID NO: 8 SB9(ROS) nucleic acid, codon-optimized
SEQ ID NO: 9 SB9(CAS) nucleic acid, codon-optimized
SEQ ID NO: 10 SB9(ROSCAS) nucleic acid, codon-optimized
SEQ ID NO: 11 SB9(NEG) nucleic acid, codon-optimized
SEQ ID NO: 12 truncated EGFR, amino acids
SEQ ID NO: 13 P2A, amino acids
SEQ ID NO: 14 EF1a promotor and SB9(WT) (codon-optimized)

### Detailed description of the drawings

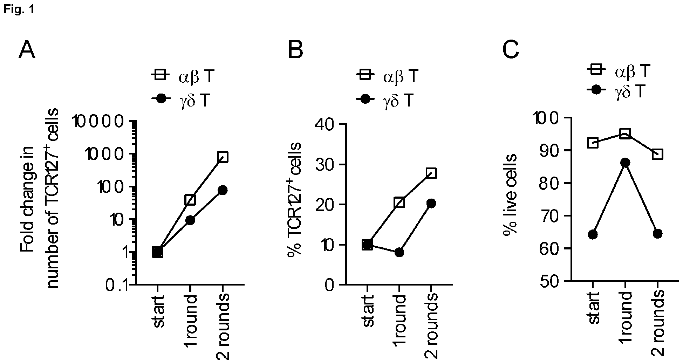

Fig. 1. γδ T cells exhibit significantly poorer proliferation and viability in response to antigen recall compared to αβ T cells. T cells were expanded *in vitro* and transduced with a construct encoding an HLA-A*01:01-restricted αβTCR (TCR127) and a CD8 co-receptor. T cells were then subjected to 2 rounds of recall stimulations with cognate antigen-expressing cancer cells, with a 6-7 day interval between stimulations. After each round of stimulation, cell numbers, viability and TCR127 expression were assessed by flow cytometry. A) The fold change in the number of antigen-specific (TCR127⁺) T cells was approximately 10-fold greater for αβT cells than for γδT cells B) Enrichment of TCR127⁺ T cells was consistently higher in αβ T cells compared to γδ T cells following both rounds of stimulation. C) The viability of αβ T cells remained high compared to γδ T cells, which showed an initial improvement after the 1^{st} recall stimulation, but declined after the second. Plots show mean values from 2 independent T cell donors.

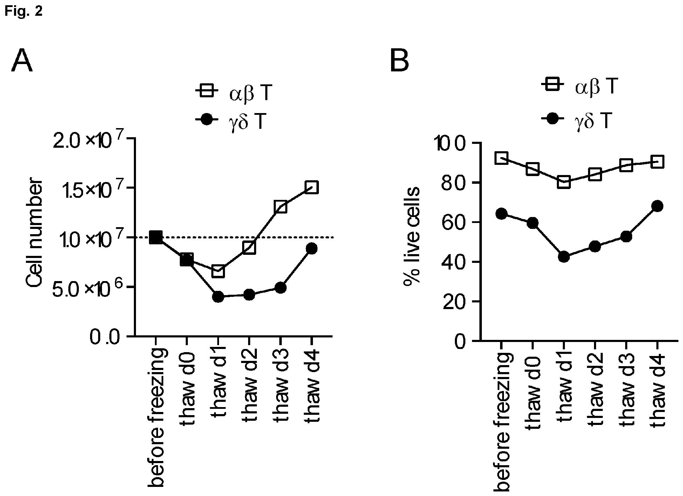

Fig. 2 αβ T cells are more resilient than γδ T cells to the stresses of cryopreservation. Engineered αβ T or γδ T were frozen at a density of 1x10⁷ cells per vial. After thawing, they were placed in 5 mL culture medium containing the cytokines IL-7 and IL-15. Daily measurements of cell number and viability were made by flow cytometry. A) Cell numbers. The αβ T cells showed a mild decrease in cell numbers on day 1 after thawing, but quickly recovered and surpassed the initial cell number of 1x10⁷ by day 3. In contrast, γδ T cells showed a stronger decline in cell numbers on day 1 after thawing, which persisted for another 2 days. By day 4, cell numbers recovered, but did not reach the initial cell number of 1x10⁷. B) Viability. The αβ T cells showed higher viability and a milder decrease on day 1 than the γδ T cells. Both populations recovered their viability by day 4. Plots show mean values from 2 independent T cell donors.

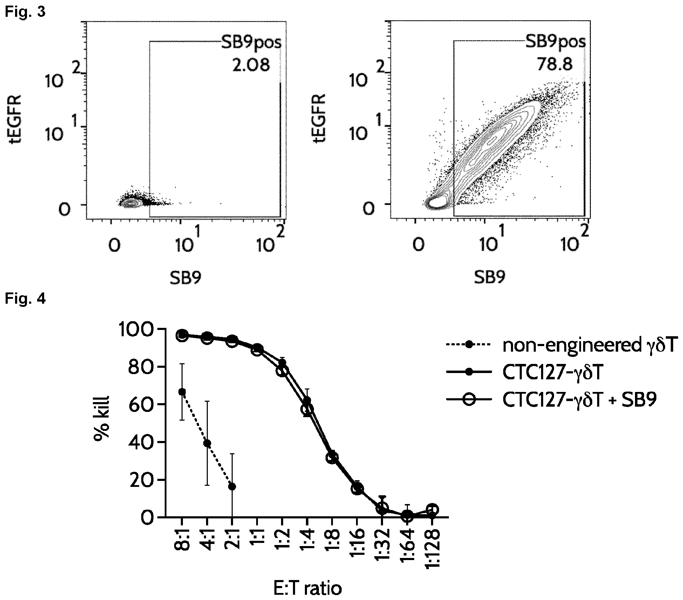

Fig. 3 Intracellular staining of SB9 on γδ T cells. γδ T cells were transduced with a construct encoding SB9 and tEGFR, or left non-engineered as controls, and cultured for 11 days. Cells were stained for EGFR on the surface prior to fixation, permeabilization and staining for SB9. The left-hand side plot shows control non-engineered γδ T cells. The right-hand side plot shows γδ T cells engineered with the SB9-tEGFR construct, which show a clear intracellular staining for SB9, concomitant to the surface staining of tEGFR.

Fig. 4 SB9 does not alter the cytotoxicity of αβTCR-γδT cells. A total of 1x10⁴ K562 target cells expressing HLA-A*01:01, full-length MAGE-A4 and firefly luciferase were mixed with decreasing numbers of effector γδ T cells expressing CTC127, with or without SB9, starting at an effector-to-target (E:T) ratio of 8:1. After overnight incubation (18 - 20 hours), the remaining live K562 cells were quantified by the addition of the luciferase substrate and measurement of light emission. Degree of killing was quantified by the formula 100 - ((signal T cells / signal targets alone) * 100). Data show mean ± SEM from 2 independent T cell donors.

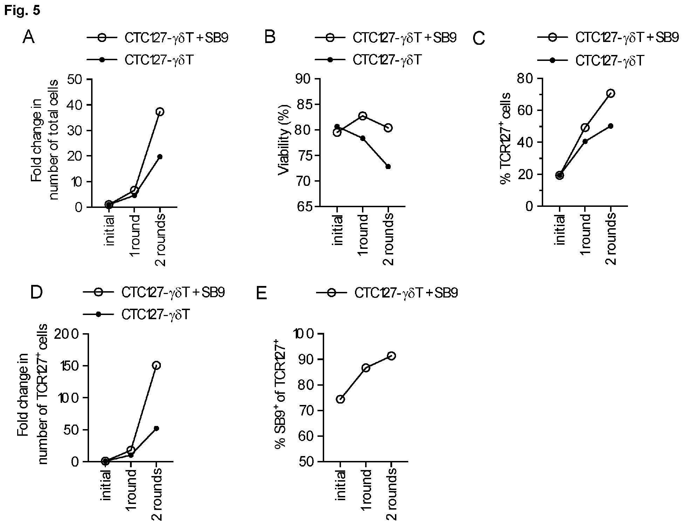

Fig. 5 SB9 confers an expansion and survival advantage to αβTCR-engineered γδT cells. γδT cells were engineered with CTC127 or with CTC127 and SB9 and expanded *in vitro* for 12 to 16 days before restimulation. For the first restimulation cycle, a total of 5x10⁵ γδ T cells (CTC127 transduction rate adjusted to 19 % - 20 %) were incubated with 2x10⁵ target cancer cells expressing their cognate HLA and antigen (K562-A01-MAGEA4). Cells were fed every 2-3 days by exchange of ½ volume of culture medium. After 6 days, cells were counted and analysed by flow cytometry (measurement of CTC127 and tEGFR expression). Fold expansion was calculated based on count after 6 days and initial cell number. For the second round of restimulation, cells from the 1^{st} round were again plated at 5x10⁵ γδ T cells with 2x10⁵ target cancer cells. Cells were fed every 2-3 days by exchange of ½ volume of culture medium. After 6-7 days, cells were counted and analysed by flow cytometry (measurement of CTC127 and tEGFR expression). Fold expansion was calculated based on count after 6-7 days and initial cell number. The cumulative fold expansion was calculated by multiplying the fold expansion after both rounds of restimulation. A) Fold expansion of total γδ T cells after restimulation cycles. B) Viability after restimulation cycles (measured by flow cytometry with a DNA-intercalating viability dye). C) Frequency of TCR127⁺ γδ T cells after restimulation cycles. D) Fold expansion of TCR127⁺ γδ T cells after restimulation cycles. E) Frequency of SB9⁺ cells (measured as tEGFR) within TCR127⁺ γδ T cells after restimulation cycles. Data show mean from 3 independent T cell donors.

### Examples

### Materials & methods

### Generation of transduced γδ T cells

Transduced gamma delta T cells were generated from PBMCs via cultivation with 1µg/ml zoledronate ²⁸. On days 3 and 4 after stimulation, cells were transduced with a retrovirus containing transgene(s), e.g. a transgenic TCR (TCR127) and coreceptor and/or an SB9. The retrovirus was generated by transfection of GALV cells with plasmid DNA of the transgene(s) using Lipofectamine transfection kit (Invitrogen) according to the manufacturer's instructions. Transduced gamma delta T cells were further cultivated for 11 to 16 days and used for assays. The expression of the transgenic TCR was assessed by flow cytometry with antibodies against the murine constant TCR beta chain.

### Generation of transduced αβ T cells

For the generation of transduced T cells, PBMCs were isolated from fresh blood via gradient centrifugation and T cells contained therein) were stimulated with bead-immobilized anti-CD3 and anti-CD28 antibodies and cultivated in RPMI medium supplemented with 10% FBS, 1 % penicillin/streptomycin, 1 mM sodium pyruvate, 0.1 mM MEM non-essential amino acids and 400 IU/mL human recombinant IL-2. On days 2 and 3 after stimulation, cells were transduced with a retrovirus containing transgene(s), e.g. a transgenic TCR (TCR127) and coreceptor. The retrovirus was generated by transfection of GALV cells with plasmid DNA of the transgene(s) using Lipofectamine transfection kit (Invitrogen) according to the manufacturer's instructions. Transduced T cells were further cultivated for 11 days and used for assays. The expression of the transgenic TCR and coreceptor was assessed by flow cytometry with antibodies against the murine constant TCR beta chain.

### Recall stimulation experiment

In order to assess expansion and enrichment of transduced TCR-T cells (αβ T and/or γδ T), an in vitro antigen-specific recall and expansion assay was developed. For the 1^{st} round of recall stimulation, a 5x10⁵ cells from a T cell suspension containing 10 % - 20 % of T cells transduced with a TCR, e.g. TCR127, with or without SB9, were placed in culture together with 2x10⁵ cancer cells expressing the cognate antigen and HLA of the transduced TCR (e.g. for TCR127: MAGEA4 on HLA-A*01:01). The cells were cultured for 6-7 days, with medium exchange every 2-3 days. After this round of stimulation, cell numbers, viability and TCR127 expression (via staining of the murine TCR beta constant chain) were assessed by flow cytometry. Fold expansion was calculated by dividing the cell number after stimulation by the initial cell number. A second round of stimulation was then initiated with 5x10⁵ T cells and 2x10⁵ antigen-expressing cancer cells. The cells were cultured for 6-7 days, with medium exchange every 2-3 days. After this round of stimulation, cell numbers, viability and TCR127 expression were again assessed. Fold expansion of the second recall stimulation was calculated by dividing the cell number after stimulation by the initial cell number. The cumulative fold expansion was calculated by multiplying the fold expansions after the 1^{st} and 2^{nd} stimulations. The expansion of TCR⁺ cells was calculated based on the total cell counts and the frequency of TCR⁺ cells in the samples.

### Freeze-Thaw experiment

Engineered αβ T or γδ T were frozen at a density of 1x10⁷ cells per vial and kept in liquid nitrogen until thawing. After thawing, they were placed in 5 mL culture medium containing the cytokines IL-7 and IL-15. Daily measurements of cell number and viability were made by flow cytometry

### Intracellular staining of SB9

T cells were stained for surface markers (e.g. EGFR) with titrated amounts of fluorophore-labelled antibodies for 10 minutes at room temperature. After washing, cells were fixed with a 4 % paraformaldehyde solution in PBS for 20 minutes at room temperature. Cells were then washed twice with 1X Flow Cytometry Perm Buffer (Proteintech). Cells were then stained with a titrated amount of a fluorophore-labelled monoclonal antibody against human SB9 (Proteintech, clone 2H9D2) for 20 minutes at room temperature. Cells were then washed twice with 1X Flow Cytometry Perm Buffer, resuspended in PBS and acquired on a flow cytometer.

### Kill assay

For the killing assay, a fixed number (1x10⁴) of K562 cells expressing HLA-A*01:01, MAGE-A4 and luciferase were used as target cells. Effector T cells were added in different effector-to-target (E:T) ratios. After 18-20 hours, the luciferase activity of the remaining live K562 cells were measured and the degree of lysis by the T cells calculated with the formula: 100 - [(signal with T cells) / (signal without T cells) * 100]

### Results

### Recall stimulation

To evaluate the proliferative capacity of T cells in response to antigen recall stimulations, we transduced αβ T cells and γδ T cells from the same donor with a tumor antigen-specific T-cell receptor (TCR). The transduced T cells were then subjected to two rounds of recall stimulations using a cancer cell line expressing the cognate antigen. Prior to the first stimulation, the T-cell suspension was adjusted to contain 10% tumor-specific TCR-expressing T cells, as determined by measuring the mCb tag (murine TCR beta constant chain) encoded by the transgenic TCR. Each round of stimulation was conducted over a 6- to 7-day period, after which the cells were counted, and mCb expression was analyzed to assess the number and enrichment of antigen-specific T cells.

Our results revealed that the enrichment of mCb⁺ T cells was consistently higher in αβ T cells compared to γδ T cells following both rounds of stimulation (**Fig**. **1B**). Specifically, the fold change in the number of antigen-specific T cells was approximately 10-fold greater for αβ T cells than for γδT cells (**Fig. 1A**). Additionally, the viability of γδ T cells after the 2 recall rounds was lower than that of αβ T cells (Fig. 1C). These findings indicate that γδ T cells exhibit significantly poorer proliferation in response to antigen recall compared to αβ T cells.

Repeated antigenic stimulations are known to upregulate granzyme B production, which can leak from cytolytic granules into the cytoplasm, potentially inducing cell death. The reduced proliferation as shown may be attributed to an increased susceptibility of γδT cells to activation-induced cell death (AICD) and/or an inherently lower proliferative capacity. Still, overexpression of Serpin B9 in γδT cells can mitigate AICD by neutralizing endogenous granzyme B activity, thereby improving their survival and proliferative response under these conditions.

### Cryopreservation

We assessed the ability of αβ T cells and γδ T cells to withstand cryopreservation by freezing 1 x 10⁷ cells, storing them for 3 days, thawing, and then culturing them post-thaw. Cell number and viability were measured daily for 4 days after thawing. Both αβ T and γδ T cells exhibited a decline in viability and cell numbers on day 1 post-thaw, with the decline being more pronounced in γδT cells compared to αβT cells. By day 2, αβ T cells had fully recovered and began to expand, surpassing their initial cell count of 1 × 10⁷ by day 3. In contrast, γδ T cells did not show significant recovery in viability or cell numbers until day 4 (Fig. 2).

These findings demonstrate that αβ T cells are more resilient than γδ T cells to the stresses of cryopreservation.

SerpinB9 can be stained in engineered γδ T cells.

In order to assess whether transduction of γδ T cells with SB9 can increase their SB9 protein content, we performed a direct intracellular staining of SB9 using a monoclonal antibody (clone 2H9D2). Non-engineered γδ T cells showed very little staining of SB9, suggesting that endogenous SB9 levels are below the detection limit of the assay (Fig 3, left-hand side panel). In contrast, γδ T cells transduced with a construct encoding SB9 and a truncated EGFR (as a surface marker) showed a clear and concomitant staining for both proteins (Fig 3, right-hand side panel). These data indicate that the intracellular levels of SB9 in γδ T cells can be increased by genetically engineering the cells with a construct encoding SB9.

SerpinB9 does not interfere with the cytotoxicity of TCR-γδT cells.

Because SB9 is a potent inhibitor of granzyme B, we tested whether its overexpression reduces the cytotoxicity of TCR-engineered γδ T cells. We engineered γδ T cells with CTC127, an affinity-matured αβTCR specific for a MAGE-A4 epitope presented by HLA-A*01:01, a CD8 co-receptor and a TGFβ switch receptor. Additionally, we transduced the γδ T cells with a construct encoding SB9 and truncated EGFR (tEGFR) as a surface marker in order to assess transduction rates by flow cytometry. We then used CTC127 or CTC127+SB9 in a kill assay against K562 cancer cells engineered to express HLA-A*01:01, the full-length MAGE-A4 gene and firefly luciferase (K562-A01-MAGEA4). We observed a similar killing curve for both CTC127 and CTC127+SB9 γδ T cells (Fig. 4), leading to the conclusion that overexpression of SB9 did not inactivate intracellular granzyme B and thus did not reduce the cytotoxic capacity of the engineered γδ T cells. This observation confirms that granzyme B and SB9 are physically separated inside the cells, the former is inside of granules and the latter is in the cytoplasm, so that granzyme B is not affected by SB9 overexpression.

Serpin B9 protects TCR-γδT cells from activation-induced cell death and promotes their expansion and enrichment.

Activation-induced cell death (AICD) is the programmed cell death (apoptosis) of immune cells that occurs after stimulation (single and/or repeated) with cognate ligands. It is a physiological mechanism taking place to restrict immune cell numbers, e.g. to establish peripheral tolerance or during the contraction phase after an immune response. The exact mechanism(s) of AICD are still not fully elucidated. Apoptosis induction via FasL-Fas interactions or granzyme B leakage into the T cell's cytoplasm are proposed ¹⁶⁻¹⁹. In the context of T cell manufacturing for therapeutic applications, AICD can limit the cellular yield in the final product, especially if the T cells are restimulated during the process, e.g. to enrich for receptor-expressing T cells. Additionally, γδ T cells are particularly sensitive to AICD ^{5,6}, which can not only limit their *in vitro* expansion potential, but also their survival, efficacy and persistence *in vivo* upon antigen encounter.

To address this challenge, we assessed whether overexpression of SB9 can protect αβTCR-γδT cells from AICD after repeated stimulations *in vitro.* We generated γδ T cells expressing CTC127 either alone or with SB9 and submitted them to 2 rounds of restimulation (6-7 days apart) with cancer cells expressing their cognate antigen (K562-A01-MAGEA4). At the start of the experiment, we adjusted the frequency of CTC127⁺ γδ T cells to 19 % - 20 %, so that all samples have the same initial number of CTC127-expressing γδ T cells. At the end of each restimulation round, we counted the cells, assessed their viability and their expression of the CTC127 αβTCR (via staining of the murine TCR beta constant chain) and SB9 (via staining of tEGFR). We observed that γδ T cells expressing SB9 expanded more than the control γδ T cells after both rounds of antigen-specific restimulation. γδ T cells expressing SB9 showed a 40-fold expansion, while control γδ T cells showed only a 20-fold expansion (**Fig. 5A**)**.** SB9 also improved the viability of the γδ T cells throughout the whole restimulation cycles, whereas γδ T cells not expressing SB9 slightly lost viability (Fig. 5B).

We next measured the expression of the transduced αβTCR TCR127 by the γδ T cells after the restimulation cycles in order to assess whether γδ T cells expressing the αβTCR were enriched via the antigen-specific restimulation. We observed that both rounds of restimulation led to increase in the frequence of TCR127⁺ cells in the samples indicating that antigen drove the specific expansion of T cells expressing the cognate TCR (**Fig. 5C**). The enrichment was greater in the cells engineered with SB9 which reached on average 70 %, while the sample without SB9 reached only up to 50 % (3.5-fold compared to 2.5-fold increase, respectively). Based on the total cell counts and the frequency of TCR127⁺, we calculated the fold change in the number of antigen-specific (TCR127⁺) γδ T cells in each sample. The fold expansion in cell number of control TCR127⁺ γδ T cells after 2 rounds of antigen-specific stimulation was on average 50-fold. In contrast, SB9 expression enabled a 150-fold expansion of TCR127⁺ γδ T cells (**Fig. 5D**). These data demonstrate that SB9 expression provides a substantial advantage to γδ T cells and allows for efficient proliferation and survival after recall, likely via a reduction in granzyme-β-mediated AICD. Furthermore, the frequency of cells expressing SB9 within the TCR127⁺ cells also increased throughout the culture period, highlighting the improved "robustness" of SB9-expressing cells (**Fig. 5E**)**.**

Together, these data suggest that CTC127 γδT cells overexpressing SB9 have a survival advantage and withstand repeated stimulation cycles resulting in their specific expansion and enrichment in the cell product. This effect is likely due to lower rates of AICD.

Another explanation for this observation could be that SB9 overexpression leads to a different memory phenotype of T cells, for example, naïve and central-memory T cells have longer survival and proliferation potential than effector-memory and Temra cells. To address this hypothesis, we stained the T cells at the end of the restimulation cycles using the markers CD27 and CD45RA to characterize their phenotype as naïve (CD27⁺CD45RA⁺), central-memory (CD27⁺CD45RA⁻), effector-memory (CD27⁻CD45RA⁻) or Temra (CD27⁻CD45RA⁺). We observed that neither SB9 overexpression nor restimulation led to differences in the phenotype of the γδT cells (data not shown). Thus, the survival and expansion advantage of SB9 overexpressing cells cannot be attributed to a shift to a less differentiated phenotype.

SerpinB9 enhances the resistance of TCR-γδT cells to freeze-thaw cycle.

True allogeneic off-the-shelf cell products require cryopreservation for scalability. Therapeutic cells have to withstand the freeze-thaw cycle with sufficient viability and cell numbers to perform their function after infusion into a patient. Poor recovery and viability after thawing is a challenge for therapeutic immune cells, including TCR- and CAR-engineered T cells. In particular, NK cells and γδT cells are very sensitive to cryopreservation ^{7,15,20}. Recent studies show that apoptosis of NK cells after a freeze-thaw cycle is due to leakage of granzyme B from their own cytolytic granules ¹⁵. We thus test whether overexpression of SB9 protects αβTCR-γδT cells from cell death after thawing.

### SerpinB9 protects γδ T cells from killing by antigen-specific T cells

Immune rejection of therapeutic allogeneic immune cells by the host's immune system can be an obstacle to achieving durable responses. Several strategies are being developed to cloak and/or improve the resistance of therapeutic allogeneic cells from killing by the host's T and NK cells (reviewed in reference ⁸). Killing mediated by perforin and granzyme B is a major mechanism of allograft rejection ²¹⁻²³. Thus, we hypothesized that overexpression of SB9 can protect allogeneic γδT cells from killing by host T cells.

To this end, we designed an *in vitro* killing assay where the target cells are γδ T cells transduced with a model antigen coupled to firefly luciferase as a reporter system and, optionally, with SB9. The effector cells are αβ T cells transduced with a TCR specific to the model antigen expressed by the target γδ T cells.

### Conclusion

Here we show that overexpression of the granzyme B inhibitor SB9 by γδ T cells can address several challenges in allogeneic cell therapies against cancer. SB9 improved γδ T expansion and survival *in vitro* upon restimulation, which can translate into higher yields during manufacturing and better *in vivo* efficacy when γδ T cells are exposed to recurrent antigenic exposure in solid tumors. Importantly, SB9 did not reduce the cytolytic capacity of γδ T cells against tumor cell targets.

Variants of SB9 which alter its function have been published. Of particular interest is the variant with cysteine to serine substitutions in the adjacent positions 341 and 342. This variant maintains its granzyme B inhibitory activity, but is resistant to inactivation by reactive oxygen species, leading to improved function under oxidative stress ²⁴. Another variant of interest contains a glutamic to aspartic acid substitution in its catalytic center, position 340 (P1 residue). This E340D variant has lower affinity to granzyme B, but higher affinity to caspases (1, 2, 5, 6, 7, 8, and 10) and protects against Fas-mediated apoptosis ²⁵. Of particular interest is a variant containing all three amino acid substitutions (E340D, C341S, and C342S), which has not been described anywhere else. We will investigate whether any of these variants can improve upon the positive effects of wild-type SB9 observed here for engineered γδ T cells.

Allogeneic off-the-shelf cell therapies hold promise to revolutionize cancer treatment in terms of scalability, accessibility and cost-effectiveness. Yet, important challenges in manufacturing, cryopreservation and *in vivo* persistence (or immunogenicity) need to be addressed in order to fulfill such promise. Overexpression of SB9 in γδ T cells, e.g., in αβTCR-engineered or CAR-engineered γδ T cells can tackle these obstacles and enable robust and effective allogeneic γδ T cells that can be used, e.g., for the therapy of solid tumors.

### References

1. Yazdanifar, M., Barbarito, G., Bertaina, A. & Airoldi, I. γδ T Cells: The Ideal Tool for Cancer Immunotherapy. Cells 9, (2020).
2. Vantourout, P. & Hayday, A. Six-of-the-best: unique contributions of γδ T cells to immunology. Nat Rev Immunol 13, 88-100 (2013).
3. Saura-Esteller, J. et al. Gamma Delta T-Cell Based Cancer Immunotherapy: Past-Present-Future. Front Immunol 13, 915837 (2022).
4. Neelapu, S. S. et al. A phase 1 study of ADI-001: Anti-CD20 CAR-engineered allogeneic gamma delta (γδ) T cells in adults with B-cell malignancies. Journal of Clinical Oncology 40, 7509 (2022).
5. Kabelitz, D. et al. Activation and activation-driven death of human gamma/delta T cells. Immunol Rev 120, 71-88 (1991).
6. Siegers, G. M. & Lamb, L. S. Cytotoxic and regulatory properties of circulating Vδ1+ γδ t cells: A new player on the cell therapy field? Molecular Therapy vol. 22 1416-1422 Preprint at https://doi.org/10.1038/mt.2014.104 (2014).
7. Burnham, R. E. et al. Human serum albumin and chromatin condensation rescue ex vivo expanded γδ T cells from the effects of cryopreservation. Cryobiology 99, 78-87 (2021).
8. Martin, K. E., Hammer, Q., Perica, K., Sadelain, M. & Malmberg, K.-J. Engineering immune-evasive allogeneic cellular immunotherapies. Nat Rev Immunol (2024) doi:10.1038/s41577-024-01022-8.
9. Zhang, M. et al. Serine Protease Inhibitor 6 Protects Cytotoxic T Cells from Self-Inflicted Injury by Ensuring the Integrity of Cytotoxic Granules. Immunity 24, 451-461 (2006).
10. Ashton-Rickardt, P. G. Serine protease inhibitors and cytotoxic T lymphocytes. Immunological Reviews vol. 235 147-158 Preprint at https://doi.org/10.111 1/j.0105-2896.2010.00892.x (2010).
11. Ashton-Rickardt, P. G. An emerging role for Serine Protease Inhibitors in T lymphocyte immunity and beyond. Immunology Letters vol. 152 65-76 Preprint at https://doi.org/10.1016/j.im-let.2013.04.004 (2013).
12. Bird, C. H. et al. The granzyme B-Serpinb9 axis controls the fate of lymphocytes after lyso-somal stress. Cell Death Differ 21, 876-887 (2014).
13. Kaiserman, D. & Bird, P. I. Control of granzymes by serpins. Cell Death and Differentiation vol. 17 586-595 Preprint at https://doi.org/10.1038/cdd.2009.169 (2010).
14. Bots, M. & Medema, J. P. Serpins in T cell immunity. J Leukoc Biol 84, 1238-1247 (2008).
15. Berjis, A. et al. Pretreatment with IL-15 and IL-18 rescues natural killer cells from granzyme B-mediated apoptosis after cryopreservation. Nat Commun 15, (2024).
16. Arakaki, R., Yamada, A., Kudo, Y., Hayashi, Y. & Ishimaru, N. Mechanism of activation-induced cell death of T cells and regulation of FasL expression. Crit Rev Immunol 34, 301-14 (2014).
17. Maher, S., Toomey, D., Condron, C. & Bouchier-Hayes, D. Activation-induced cell death: the controversial role of Fas and Fas ligand in immune privilege and tumour counterattack. Immunol Cell Biol 80, 131-7 (2002).
18. Laforge, M. et al. Apoptotic Death Concurrent with CD3 Stimulation in Primary Human CD8 T Lymphocytes: A Role for Endogenous Granzyme B 1. The Journal of Immunology vol. 176 http://journals.aai.org/jimmunol/article-pdf/176/7/3966/1217426/3966.pdf (2006).
19. Ida, H. et al. Granzyme B leakage-induced cell death: A new type of activation-induced natural killer cell death. Eur J Immunol 33, 3284-3292 (2003).
20. Szmania, S. et al. Ex vivo-expanded natural killer cells demonstrate robust proliferation in vivo in high-risk relapsed multiple myeloma patients. J Immunother 38, 24-36 (2015).
21. Carnel, N., Lancia, H. H., Guinier, C. & Benichou, G. Pathways of Antigen Recognition by T Cells in Allograft Rejection. Transplantation 107, 827-837 (2023).
22. Choy, J. C. Granzymes and perforin in solid organ transplant rejection. Cell Death Differ 17, 567-576 (2010).
23. Pa 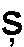 atu-Cornea, A.-M., Ciciu, E. & Tu 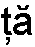 , L.-A. Perforin: An intriguing protein in allograft rejection immunology (Review). *Exp Ther Med* **24,** 519 (2022).
24. Mangan, M. S. J. et al. A novel serpin regulatory mechanism : SerpinB9 is reversibly inhibited by vicinal disulfide bond formation in the reactive center loop. Journal of Biological Chemistry 291, 3626-3638 (2016).
25. Bird, C. H. et al. Selective Regulation of Apoptosis: the Cytotoxic Lymphocyte Serpin Proteinase Inhibitor 9 Protects against Granzyme B-Mediated Apoptosis without Perturbing the Fas Cell Death Pathway. Mol Cell Biol 18, 6387-6398 (1998).
26. Teo, P.Y. et al. Overexpression of an Engineered SERPINB9 Enhances Allogeneic T-cell Persistence and Efficacy. Cancer Immunol Res 12:1108-22 (2024).
27. Tomasik J. et al. Next generations of CAR-T cells - new therapeutic opportunities in hema-tolgy? Front. Immunol 13, 1034707 (2022).
28. Tan, W.K. et al. Minireview Open Access Expansion of Gamma Delta T Cells - A Short Review on Bisphospho-nate and K562-Based Methods Gamma Delta T cells As the Next Generation of Effector Cells for Immunotherapy" J. Immunol. Sci 2(3): 6-12 (2018).
29. Verkerk T.et al. Isolation and expansion of pure and functional γδ T cells. Front. Immunol. 15:1336870 (2024).
30. Inniss, M.C. et al., Carbonic anhydrase 2-derived drug-responsive domain regulates membrane-bound cytokine expression and function in engineered T cells. Communications Biology 8: 28 (2025).
WO 2022/243514 A1 , WO 2025/027111 A1, WO 2024/042202 A1, US 2003219463 A1, WO 2019/104269 A1

## Claims

1. A γδ T cell comprising a nucleic acid encoding SerpinB9, wherein the SerpinB9 has at least 80% sequence identity to wt human SerpinB9 of SEQ ID NO: 1 and is capable of reducing granzyme B activity, and wherein the γδ T cell expresses said SerpinB9, wherein the nucleic acid encoding SerpinB9 is operably linked to a heterologous promotor and is DNA or wherein the nucleic acid encoding SerpinB9 is an mRNA having a heterologous cap.

2. The γδ T cell of claim 1, wherein the SerpinB9 is wt human SerpinB9 of SEQ ID NO: 1.

3. The γδ T cell of claim 1, wherein the SerpinB9 has C341S and C342S substitutions and is resistant to inactivation by reactive oxygen species.

4. The γδ T cell of any of claims 1 or 3, wherein the SerpinB9 has a E340D substitution and has increased affinity to a caspase.

5. The γδ T cell of any of the preceding claims, wherein the nucleic acid encoding SerpinB9 is operably linked to a heterologous promotor, wherein the promotor is selected from the group comprising human EF1α, PGK, beta actin, IF4A1, GAPDH, GRP78, HSP70, beta kinesin, ubiquitin B, CD45, MPSV, CMV promotor, or LTR promotor.

6. The γδ T cell of any of claims 1-4, wherein the nucleic acid encoding SerpinB9 is an mRNA having a heterologous cap, wherein the heterologous cap is selected from the group comprising an anti-reverse cap analog (ARCA), a two-headed cap analog a locked nucleic acid modified cap or a cap comprising a chemical modification of the phosphate bridge comprising a tetraphosphate structure and/or a substitution of O by S.

7. The γδ T cell of any of the preceding claims, further expressing an αβTCR construct and a CD8 or CD4 co-receptor, preferably, a CD8 co-receptor.

8. The γδ T cell of any of claims 1-6, further expressing a CAR.

9. The γδ T cell of any of the preceding claims, further expressing a kill switch, optionally expressing EGFR deficient in signaling on its surface.

10. The γδ T cell of any of the preceding claims wherein expression or stabilization of SB9 can be regulated.

11. The γδ T cell of any of the preceding claims, further expressing a SWITCH receptor, wherein the SWITCH receptor is capable of transforming a signal provided by TGF-β into a co-stimulatory signal.

12. A composition comprising the γδ T cell of any of the preceding claims and a suitable solvent, wherein the composition is frozen.

13. The γδ T cell of any of claims 1-11 or the composition of claim 12 for use in treating a subject having a cancer or an infection, preferably, a cancer, by adoptive T cell therapy, wherein the γδ T cell is an allogeneic cell,
wherein, if the T cell is in a frozen composition, the composition is thawed before administration to the subject.

14. A method comprising transfecting a γδ T cell with a nucleic acid encoding SerpinB9, thus generating a γδ T cell of any of claims 1-11, wherein the method is
a) a method of increasing cell viability of a γδ T cell after freezing; and/or
b) a method of increasing cell viability after γδ T cell activation; and/or
c) a method for reducing immune rejection of γδ T cells after allogeneic transfer.

15. A method of preparing a γδ T cell composition, comprising
a) providing a γδ T cell,
b) transfecting the γδ T cell with a nucleic acid encoding SerpinB9, thus generating a γδ T cell of any of claims 1-11,
c) freezing a composition comprising said T cell,
d) thawing the composition comprising said T cell, and
e) formulating the composition for administration to a human subject,
wherein, optionally, the method further comprises restimulating the T cell composition.
